Europäisches Patentamt

European Patent Office

Office européen des brevets

(11) Veröffentlichungsnummer: **0 419 729 A1**

## (12) EUROPÄISCHE PATENTANMELDUNG

(21) Anmeldenummer: 89118096.0

(22) Anmeldetag: 29.09.89

(51) Int. Cl.⁵: **A61B 5/06, A61B 8/08**

Die Bezeichnung der Erfindung wurde geändert (Richtlinien für die Prüfung im EPA, A-III, 7.3).

(43) Veröffentlichungstag der Anmeldung:
**03.04.91 Patentblatt 91/14**

(84) Benannte Vertragsstaaten:
**DE FR GB**

(71) Anmelder: **Siemens Aktiengesellschaft**

Wittelsbacherplatz 2
**W-8000 München 2(DE)**

(72) Erfinder: **Ermert, Helmut, Prof.-Dr.-Ing.**
**Eichenring 15**
**W-8551 Röttenbach(DE)**
Erfinder: **Pfeiler, Manfred, Dr.-Ing.**
**Ludwig-Thoma-Strasse 9**
**W-8520 Erlangen(DE)**

(54) **Ortung eines Katheters mittels nichtionisierender Felder.**

(57) Zur Ortung eines Katheters (2) in einem Gefäß (1) ist die Katheterspitze (3) mit einem Sender (4) für elektromagnetische oder akustische Felder oder Wellen ausgerüstet. Ein Empfänger (5) erfaßt diese Wellen mittels an den Körper angekoppelter Sensoren bzw. Antennen (8a, b, c ...) und wandelt sie in elektrische Ortungs-oder Bildsignale um. Zum Zwek-ke der Lokalisierung der Katheterspitze in bezug auf die Gefäßarchitektur werden die Ortungssignale mit einem auf andere Weise (z.B. Röntgen) gewonnenen Bild der Gefäßarchitektur in einem Sichtgerät (6) kombiniert dargestellt.

EP 0 419 729 A1

# VERFAHREN ZUR ECHTZEITDARSTELLUNG EINES KATHETERS UND KATHETER ZUR DURCHFÜHRUNG DES VERFAHRENS

Bei angiographischen Untersuchungen und insbesondere in der interventionellen Technik arbeitet man innerhalb der Anwendung der Röntgendurchleuchtung sehr häufig mit der sogenannten Pfadfinderdarstellung. Dabei wird zunächst zur Ermittlung der genauen Topologie eines interessanten Gefäßbereiches (Gefäßarchitektur) mittels Kontrastmitteltechnik das Röntgenbild des Gefäßbaumes aufgenommen und gespeichert. Bei der nachfolgenden Anwendung eines Katheters wird letzterer nun in Durchleuchtungsbildern, in denen die Gefäße nicht sichtbar sind, dargestellt. Die Durchleuchtungsbildfolgen, die die Position, Orientierung und die Bewegung des Katheters wiedergegeben, werden dabei ständig mit dem die Gefäßarchitektur enthaltenden Kontrastmittelbild kombiniert (überlagert). Die resultierenden Gesamtbildfolgen zeigen demgemäß den Katheter innerhalb der Gefäßarchitektur und erlauben somit seine kontrollierte Bewegung durch den Arzt.

Diese Technik macht die Anwendung einer Durchleuchtungseinrichtung sowohl bei der Kontrastmittelinjektion zur Erzeugung des Gefäßbaumspeicherbildes als auch während der nachfolgenden Katheteranwendung notwendig. Dieses bedeutet eine Strahlenexposition für den Patienten und besonders für den Arzt, die größer ist als für die Erzeugung des Gefäßbaumspeicherbildes erforderlich.

Für die Aufnahme und Speicherung der Gefäßarchitektur ist die Röntgentechnik mit in der Regel über den Katheter erfolgender Kontrastmittelanwendung konkurrenzlos. Die Aufnahme erfolgt einmalig, und zwar vor der weiteren Katheteranwendung. Sie bedeutet für den Patienten und für den Arzt nur eine geringe Strahlenexposition.

Es ist Aufgabe der Erfindung, für die permanente Darstellung des Katheters während der Behandlung die Röntgendurchleuchtung durch andere Verfahren zu ersetzen, die von nichtionisierenden Strahlen, Wellen oder Feldern Gebrauch machen und damit den Vorteil der entfallenden Strahlenexposition aufweisen.

Diese Aufgabe ist bei einem Verfahren zur Echtzeitdarstellung eines Katheters in einem Gefäß gelöst durch einen Sender für elektromagnetische oder akustische Wellen, die von einem Empfänger erfaßt und in elektrische Bildsignale umgewandelt werden, wobei der Sender oder Empfänger dem Katheter zugeordnet ist.

Es sind prinzipiell folgende Möglichkeiten zu unterscheiden:

1. Die bekannte Technik erlaubt eine technisch einfache Darstellung des Katheters, da sich dieser von seiner Umgebung (biologisches Gewebe) in bezug auf die Wechselwirkung mit den angewendeten Strahlen (Röntgenstrahlen) stark unterscheidet (Kontrast). Mit einem Abbildungsverfahren und einem entsprechenden echtzeitfähigen Abbildungssystem, das von nichtionisierender Strahlung Gebrauch macht und bei dem ähnlich gute oder noch günstigere Kontrastverhältnisse vorliegen (Ultraschall, Mikrowellen), kann die Pfadfindertechnik im Prinzip ebenfalls angewendet werden, indem das den Katheter darstellende Nicht-Röntgen-Bild mit dem Kontrastmittelbild in entsprechender Weise kombiniert wird. Damit ist das oben beschriebene Problem ausgeräumt. Allerdings sind die Anforderungen an das alternative Abbildungssystem hinsichtlich seiner Applizierbarkeit und Auflösung recht hoch.

2. Eine weitere Möglichkeit besteht in der Anwendung eines Ortungsverfahrens. Ortungsverfahren unterscheiden sich in folgender Weise von Abbildungsverfahren: Abbildungsverfahren sind in der Lage, mehrere Objekte oder Objektpunkte innerhalb eines Bildes im Rahmen bestimmter Grenzen topologisch korrekt darzustellen und aufzulösen. Diese Eigenschaft, die in der Radartechnik mit Mehrzielfähigkeit bezeichnet wird, besitzen die Ortungsverfahren nicht. Letztere arbeiten also nur in dem Fall genau und eindeutig, in dem nur ein Objekt darzustellen, d.h. zu orten ist. Der Katheter ist nun eigentlich kein singuläres Ziel, seine vollständige Abbildung erfordert ebenfalls ein geeignetes, mehrzielfähiges Abbildungsverfahren. In der Pfadfindertechnik ist aber durch die Verfügbarkeit einer topographischen Darstellung der Gefäßarchitektur die vorgegebene Bewegungsrichtung des Katheters bekannt. Falls es nun gelingt, einen singulären und geeigneten Objektpunkt, z.B. die Spitze des Katheters, permanent zu orten, aufeinanderfolgende Positionen zu speichern und damit auch die Bewegung dieses Punktes zu verfolgen, so ist damit sämtliche Information verfügbar, die zu einer Rekonstruktion und bildhaften Darstellung des Katheters im unterlegten Kontrastmittelbild notwendig ist. Der Vorteil eines Ortungsverfahrens gegenüber einem Abbildungsverfahren besteht nun darin, daß mit Wellenfeldern gearbeitet werden kann, bei denen die verwendete Wellenlänge, die jeweils durch die Frequenz und die Phasengeschwindigkeit des umgebenden Mediums (Gewebe) festgelegt ist, relativ groß sein darf und nicht in der Größenordnung der Ortungsgenauigkeit liegen muß.

Bei den in Frage kommenden nichtionisierenden Wellenarten (elektromagnetische Wellen, akustische Wellen) nimmt bekanntlich die Reichweite mit zunehmender Frequenz stark ab. Somit ist die Möglichkeit, bei Anwendung eines Ortungsverfahrens relativ große Wellenlängen und damit geringere Frequenzen anzuwenden, von Vorteil. Weiterhin ist der Aufwand an Signalbandbreite und Apertur, insbesondere hinsichtlich ihrer spektralen (Signal) bzw. spatialen (Apertur) Belegungsdichte bei Ortungsverfahren sehr viel geringer als bei Abbildungsverfahren. Es reicht aus, den zu ortenden Objektpunkt bei wenigen diskreten Frequenzen (z.B. 3 bis 5) mit wenigen extrakorporalen Aperturstützpunkten (z.B. 3 bis 5 Sender/Empfänger) in Wechselwirkung zu bringen. Mittels dieser Wechselwirkung werden durch Messung von Phasenbeziehungen oder Laufzeitbeziehungen Entfernungen oder Entfernungsdifferenzen in bezug auf die Objektposition und die verschiedenen Aperturstützpunkte bestimmt, die in ihrer Kombination eine eindeutige und genaue Postionsbestimmung (Ortung) des Objektpunktes möglich machen. Der Objektpunkt, also die Katheterspitze, muß allerdings in geeigneter Weise markiert werden. Im übrigen gelten wie in der konventionellen Pfadfindertechnik die Bedingungen, daß das Katheterbild und das Gefäßbild in richtiger räumlicher Zuordnung miteinander kombiniert werden und daß die Gefäßarchitektur sich während der Behandlung nicht verschiebt oder verformt.

3. Das unter 2. beschriebene Prinzip kann sinngemäß auch auf andere Hohlraumstrukturen des Körpers, z.B. das Gallengangsystem, angewendet werden, wie auch für Gebiete mit Strukturen, die durch eigenes Kontrastverhalten (z.B. Knochen) eine Orientierungsstruktur ergeben. Je nach Untersuchungstechnik kann dann an die Stelle des Katheters auch eine Nadel treten, deren Spitze geortet wird.

Es sind folgende technische Realisierungen denkbar:

a) Ortung mit elektromagnetischen Wellenfeldern

Die Markierung der Katheterspitze erfolgt hier vorteilhaft durch Ausrüstung mit einer Antenne, deren Zuleitung im oder am Katheter entlang zu führen ist. Es kommt eine elektrische Antenne (Dipol) oder eine magnetische Antenne (Schleife) in Frage. Ein Vorteil dieser Antennentypen ist in ihren Polarisationseigenschaften zu sehen, die Ortung und Bestimmung der Orientierung der Katheterspitze möglich machen. Die Antenne kann als Sende- oder als Empfangsantenne betrieben werden, wobei die extrakorporalen Antennen, die gegebenenfalls in geeig neter Form an der Hautoberfläche an das biologische Gewebe anzupassen sind, entsprechend als Empfangs- oder Sendeantennen fungieren. Bei Mehrwegeausbreitung zwischen Katheterspitze und Außenantennen kann durch geeignete Mehrfrequenz- oder Breitbandtechnik der direkte, d.h. kürzeste Weg zwischen den beteiligten Antennen bestimmt werden. Wegen der relativ großen dielektrischen Inhomogenität des biologischen Gewebes wird eine absolute Ortung mit diesem Verfahren nicht sehr genau sein. Falls aber die aktuelle Position der Katheterspitze zu einem Zeitpunkt oder auch mehreren Zeitpunkten kurzzeitig, z.B. mittels Durchleuchtung verfügbar gemacht wird, werden sich in der darauffolgenden Zeit oder in den zeitlichen Zwischenräumen relative Änderungen der Position mit dem elektromagnetischen Ortungsverfahren mit großer Genauigkeit bestimmen lassen.

b) Ortung mit akustischen Wellen

In entsprechender Form wird die Ortung auch mittels akustischer Wellen möglich sein. Das Problem des Kontrastes des biologischen Gewebes ist im akustischen Fall wesentlich geringer als im elektromagnetischen Fall. Das Problem von Mehrwegeausbreitung wird im Falle der akustischen Wellen aber wegen der geringeren Dämpfung größer sein. Beide Probleme sind aber in gleicher Weise wie im Falle a) beschrieben lösbar. Die Möglichkeit der Bestimmung der Orientierung der Katheterspitze ist wegen der fehlenden Polarisationseigenschaft der akustischen Wellen nicht wie im elektromagnetischen Fall gegeben. Allerdings sind ausgeprägte Richtcharakteristiken der akustischen Antennen prinzipiell für die Bestimmung der Orientierung der Katheterspitze als Träger der Antenne nutzbar. Eine Bestückung der Katheterspitze mit mehreren separaten Antennenelementen bietet eine weitere Möglichkeit zur Bestimmung der Orientierung. Technisch kommt für die Antenne am Katheter ein keramisches oder polymeres piezoelektrisches Element in Betracht. Wegen der hohen Sendesignalamplituden ist für den Fall der akustischen Ortung der Betrieb der Katheterantenne als Empfangsantenne vorteilhafter. Da die Übertragungswege reziprok zueinander sind, sind die Ortungsergebnisse bei Umkehr der Übertragungsrichtung äquivalent.

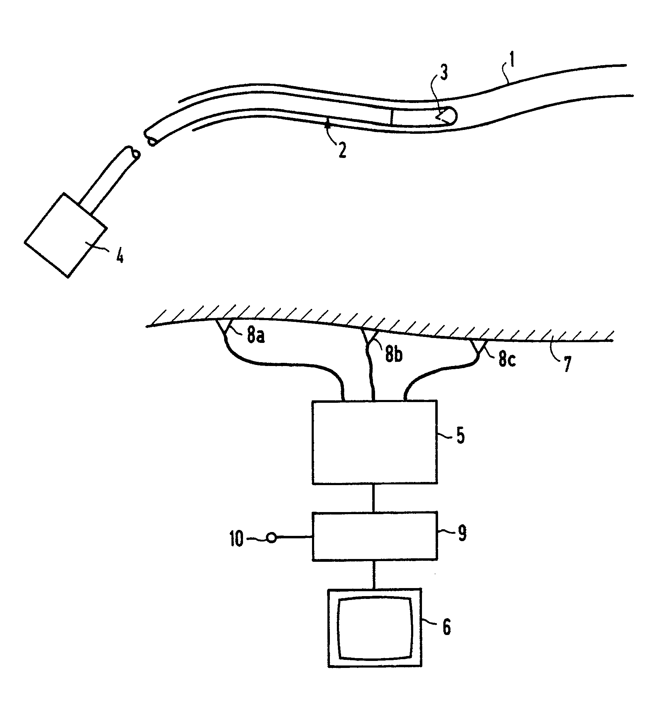

Die Erfindung ist nachfolgend anhand eines in der Zeichnung dargestellten Ausführungsbeispieles näher erläutert.

In der Zeichnung ist ein Gefäß 1 dargestellt, in das ein Katheter 2 eingeschoben ist. An seiner Spitze trägt er eine Antenne 3, die von einem Sender 4 mit Energie versorgt wird, z.B. einen Dipol.

Zur Ortung der Lage der Spitze 3 ist ein Empfänger 5 vorgesehen, der die von der Antenne 3 ausgesandten elektromagnetischen Wellen mittels Empfangsantennen 8a, 8b, 8c auf der Körperoberfläche 7 empfängt. Ein Sichtgerät 6 dient zur bildhaften Darstellung der Lage der Katheterspitze in der beschriebenen Weise, z.B. auch durch Überlagerung mit einem Röntgenbild.

Wie oben beschrieben kann an der Katheterspitze auch eine Empfangsantenne vorgesehen sein, die mit einem am äußeren Katheterende angeordneten Empfänger in Verbindung steht. Außerhalb der Körperoberfläche 7 ist dann anstelle eines Empfängers ein Sender vorhanden.

Die zu verwendenden Wellen können wie oben beschrieben auch Ultraschallwellen sein.

Zur Verarbeitung der Empfangssignale dient eine Signalverarbeitungseinheit 9, der über einen gesonderten Eingang 10 die Bildinformation bezüglich der Gefäßarchitektur zugeführt wird. Die Sendeantenne, die der Katheterspitze zugeordnet ist, kann auch durch eine Empfangsantenne oder einen Sensor ersetzt werden. In dieser Betriebsart sind 4 ein Empfänger, 5 ein Sender und 8a, b, c Sendeantennen.

Bei der beschriebenen Anordnung wird ein resultierendes bzw. fortgeschriebenes Bild der Katheterspitze erzeugt, bei dem die im zeitlichen Ablauf georteten Positionen den zurückgelegten Weg der Katheterspitze beschreiben. Der Spitzenbereich kann dabei mit individuell ortungsfähigen Segmenten versehen sein, wobei durch ein Mehrfrequenz- oder Multiplex-Verfahren Ort und Richtung der Katheterspitze feststellbar sind.

**Ansprüche**

1. Verfahren zur Echtzeitdarstellung eines Katheters (2) in einem Gefäß (1) mit einem Sender (4) für elektromagnetische oder akustische Wellen, die von einem Empfänger (5) erfaßt und in elektrische Bildsignale umgewandelt werden, wobei der Sender (4) oder Empfänger (5) dem Katheter (2) zugeordnet ist.

2. Verfahren nach Anspruch 1, **dadurch gekennzeichnet,** daß das Bild des Katheters (2) einem röntgenologischen Gefäßbild überlagert wird.

3. Verfahren nach Anspruch 1 oder 2, **dadurch gekennzeichnet,** daß ein Bild der Katheterspitze erzeugt wird.

4. Verfahren nach Anspruch 3, **dadurch gekennzeichnet,** daß ein resultierendes bzw. fortgeschriebenes Bild erzeugt wird, bei dem die im zeitlichen Verlauf georteten Positionen den zurückgelegten Weg der Katheterspitze beschreiben.

5. Katheter zur Durchführung des Verfahrens nach einem der Ansprüche 1 bis 4, **dadurch gekenn-** **zeichnet,** daß er an seiner Spitze eine Sende- oder Empfangsantenne (3) trägt.

6. Katheter nach Anspruch 5, **dadurch gekennzeichnet,** daß der Spitzenbereich des Katheters (2) mit individuell ortungsfähigen Segmenten (3) versehen ist, wobei durch ein Mehrfrequenz- oder Multiplex-Verfahren Ort und Richtung der Katheterspitze feststellbar sind.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-4697595 (BREYER ET AL) <br> * Figuren * <br> * Spalte 3, Zeile 24 - Spalte 4, Zeile 38 * | 1 | A61B5/06 <br> A61B8/08 |
| A | | 3, 5 | |
| X | MEDICAL AND BIOLOGICAL ENGINEERING AND COMPUTING. <br> vol. 22, no. 3, Mai 1984, STEVENAGE GB <br> Seiten 268 - 271; BREYER et al.: <br> "Ultrasonically marked catheter - a method for positive echographic catheter position identification." <br> * das ganze Dokument * | 1 | |
| A | | 3, 5 | |
| A | FR-A-2545349 (DURET) <br> * Figuren * <br> * Seite 3, Zeile 3 - Seite 7, Zeile 19 * | 1-4 | |
| A | EP-A-91577 (KUNKE) <br> * Figuren * <br> * Seite 3, Zeile 33 - Seite 6, Zeile 7 * <br> * Seite 10, Zeilen 7 - 19 * | 1, 5, 6 | RECHERCHIERTE SACHGEBIETE (Int. Cl.5 ) <br><br> A61B |

Der vorliegende Recherchenbericht wurde für alle Patentansprüche erstellt

| Recherchenort | Abschlußdatum der Recherche | Prüfer |
|---|---|---|
| DEN HAAG | 29 MAI 1990 | CHEN A.H. |